# EUROPEAN PATENT SPECIFICATION

(11) **EP 4 203 754 B1**
(45) Date of publication and mention of the grant of the patent: **09.09.2026**
(21) Application number: 21859406.7
(22) Date of filing: 25.08.2021
(51) Int. Cl.: A47J 37/08, G01N 21/00

(54) **HEATABLE KITCHEN DEVICE**
BEHEIZBARE KÜCHENEINRICHTUNG
DISPOSITIF DE CUISINE CHAUFFANT

(30) Priority: 28.08.2020 AU 2020903081
(43) Date of publication of application: 05.07.2023
(73) Proprietor: Breville PTY Limited, Alexandria, NSW 2015 (AU)
(72) Inventor: WHITE, Gerard, Alexandria, New South Wales 2015 (AU); MCCOLL, Nicholas, Alexandria, New South Wales 2015 (AU); PROFILIO, Alexander, Alexandria, New South Wales 2015 (AU); ZHAO, Simin, Alexandria, New South Wales 2015 (AU); CHUI, Tommy Chung Yeung, Alexandria, New South Wales 2015 (AU)
(74) Representative: Papa, Elisabetta
(86) International application number: PCT/AU2021/050961
(87) International publication number: WO 2022/040738

(56) References cited:
- WO-A1-2018/107206
- WO-A1-2018/107206
- WO-A1-2020/000056
- WO-A1-2020/000056
- US-A- 6 049 070

## Description

### Field

The present invention relates to a heatable kitchen device.

### Background

Heatable kitchen devices include toasters and ovens. A toaster including a sensor is disclosed in each of PCT Application Nos. PCT/AU2014/000941, PCT/AU2014/001155, PCT/AU2013/000809, and PCT/AU2017/000280.

PCT/AU2017/000280 discloses a toaster having a side wall with a reflective surface to reflect radiant heat towards a toasting chamber, and an external surface to which there is attached a sensor assembly. The sensor assembly provides a source beam to illuminate part of the product and receives a reflected beam that is received by a sensor, with the sensor providing a signal indicative of a property of the reflected beam and therefore a chromatic property of the product.

The toasters disclosed above experience inconsistent sensing performance due to difficulties in assembling the various elements of the toaster such that the sensor is consistently correctly seated against an inner wall of the toaster. Incorrect seating of the sensor against the inner wall of the toaster, leads to a test signal of the emitter being emitted at angles and directions that are difficult to control, thereby having a high variation in signal quality between different toaster units of the same series. Further, the sensors of the above disclosed toasters experience performance limitations due to insufficient cooling of the sensor assembly, particularly the emitters.

### Summary of Invention

It is an object of the present invention to address or overcome one or more of the above disadvantages, or at least provide a useful alternative to the above-mentioned heatable kitchen devices

The invention is defined by the appended claims.

One aspect not covered by the present invention provides a heatable kitchen device including:
a housing having an outer wall and an inner wall;
a heating area within the housing for receiving foodstuff to be heated;
a heating element located between the heating area and the inner wall;
a bracket mounted to the inner wall adjacent the heating area, the bracket including:
   a seat located opposite the inner wall, the seat receiving a sensor assembly;
   a first aperture through the bracket to the seat, the first aperture having an area; and
   a second aperture through the bracket to the seat, the second aperture having an area,
   wherein the inner wall has an opening that is larger than the combined areas of the apertures to allow the sensor assembly to detect a property of the foodstuff.

Preferably, the first and second aperture have sloped sidewalls, such that the area of the aperture changes from an interior side of the inner wall facing the foodstuff to an exterior side of the inner wall facing the bracket, wherein the sidewalls are sloped such that the area of the apertures at the interior side is larger than the area of the apertures at the exterior side.

Preferably, the bracket is manufactured from plastic material.

Preferably, the first aperture and the second aperture are adjacent and separated by a wall with a wall thickness, wherein the wall thickness is less than 3 mm, preferably less than 1.5 mm, most preferably about 1.06 mm.

Preferably, the bracket further includes:
a spacer to mount the bracket to the inner wall to create an airgap between the bracket and the inner wall.

Preferably, the housing further includes:
a lower air volume between the inner wall and the outer wall below the bracket; and
an upper air volume above the bracket,
wherein the bracket has a channel connecting the lower air volume to the upper air volume, the channel being in thermal communication with the sensor assembly.

Preferably, the upper air volume is located in the heating area and the inner wall has a second opening above the opening, the second opening connecting the heating area to the channel such that air from the channel is able to flow into the heating area and the upper air volume, thereby drawing air from the lower air volume into the channel.

Preferably, the second opening has a rounded shape.

Preferably, the upper air volume is located between the inner wall and the outer wall and the channel includes:
a channel extension at an end of the channel extending vertically into the upper air volume.

Preferably, the channel extension has two or more side walls, a first side wall being provided by the bracket and a second side wall being provided by the inner wall, so that heat is transferred from the heating area through the inner wall to the channel extension.

The sensor assembly includes:
an emitter located adjacent the first aperture so as to emit a test signal to the foodstuff in the heating area; and
a receiver located adjacent the second aperture so as to receive the test signal when reflected by the foodstuff in the heating area,
wherein the bracket includes a first rib on at least one edge of the first aperture to reduce spillage of the test signal from the first aperture.

Preferably, the bracket further includes:
a second rib on at least one edge of the second aperture to reduce spillage of an unreflected test signal to the receiver.

Preferably, the sensor assembly includes two emitters and the bracket includes two first apertures, the first apertures being located on opposite sides of the second aperture, and
wherein the second rib includes a single rib extending along an edge of each of the two first apertures and the second aperture.

Preferably, the two first apertures each have a vertical edge on a side opposite the second aperture, the vertical edge being unobstructed by the first and second rib.

Preferably, the bracket further includes:
a gasket between the seat and the sensor assembly.

Preferably, the gasket is made from silicone.

Preferably, the gasket includes an extended portion adapted to engage a hole in the sensor assembly to locate the sensor assembly relative to the gasket, and
wherein the extended portion is located to reduce interference between an emitter and a receiver of the sensor assembly.

Another aspect of the present invention provide a heatable kitchen device including:
a housing having an outer wall and an inner wall;
a heating area within the housing for receiving foodstuff to be heated;
a heating element located between the heating area and the inner wall;
a bracket mounted to the inner wall adjacent the heating area, the bracket being configured to receive a sensor assembly positioned for detecting a property of the foodstuff within the heating area;
the housing further having a lower air volume between the inner wall and the outer wall below the sensor assembly and an upper air volume above the sensor assembly; wherein,
the bracket has a channel connecting the lower air volume to the upper air volume, the channel being in thermal communication with the sensor assembly.

Preferably, the inner wall has an opening allowing the sensor assembly to detect a property of the foodstuff.

Preferably, the upper air volume is located in the heating area and the inner wall has a second opening above the opening, the second opening connecting the heating area to the channel such that air from the channel is able to flow into the heating area and the upper air volume, thereby drawing air from the lower air volume into the channel.

Preferably, the second opening has a rounded shape.

Preferably, the upper air volume is located between the inner wall and the outer wall and the channel includes:
a channel extension at an end of the channel extending vertically into the upper air volume.

Preferably, the channel extension has two or more side walls, a first side wall being provided by the bracket and a second side wall being provided by the inner wall, so that heat is transferred from the heating area through the inner wall to the channel extension.

Preferably, the bracket includes:
a seat located opposite the inner wall, the seat being configured for receiving the sensor assembly;
a first aperture through the bracket to the seat, the first aperture having an area; and
a second aperture through the bracket to the seat, the second aperture having an area,
wherein the opening in the inner wall is larger than the combined areas of the apertures to allow the sensor assembly to detect a property of the foodstuff.

Preferably, the sensor assembly includes:
an emitter located adjacent the first aperture so as to emit a test signal to the foodstuff in the heating area; and
a receiver located adjacent the second aperture so as to receive the test signal when reflected by the foodstuff in the heating area,
wherein the bracket includes a first rib on at least one edge of the first aperture to reduce spillage of the test signal from the first aperture.

Preferably, the bracket further includes:
a second rib on at least one edge of the second aperture to reduce spillage of an unreflected test signal to the receiver.

Preferably, the sensor assembly includes two emitters and the bracket includes two first apertures, the first apertures being located on opposite sides of the second aperture, and
wherein the second rib includes a single rib extending along an edge of each of the two first apertures and the second aperture.

Preferably, the two first apertures each have a vertical edge on a side opposite the second aperture, the vertical edge being unobstructed by the first and second rib.

Preferably, the bracket further includes:
a gasket between the seat and the sensor assembly, and preferably the gasket is integrated with the seat.

Preferably, the gasket includes an extended portion adapted to engage a hole in the sensor assembly to locate the sensor assembly relative to the gasket, and
wherein the extended portion is located to reduce interference between an emitter and a receiver of the sensor assembly.

### Brief Description of Drawings

Preferred embodiments of the present invention will now be described, by way of examples only, with reference to the accompanying drawings:
FIG. 1 shows a perspective view of a heatable kitchen device according to a preferred embodiment of the invention, in this case a toaster.
FIG. 2 shows a cut-away perspective view of the toaster of FIG. 1.
FIG. 3 shows a cut-away top plan view of the toaster of FIG. 1.
FIG. 4 shows a top section view of the toaster of FIG. 1.
FIG. 5 shows an exploded perspective view of the toaster of FIG. 1.
FIG. 6 shows an exploded perspective view of the toaster of FIG. 1.
FIG. 7 shows a side section view of the toaster of FIG. 1.
FIG. 8 shows a side section view of the toaster of FIG. 1.

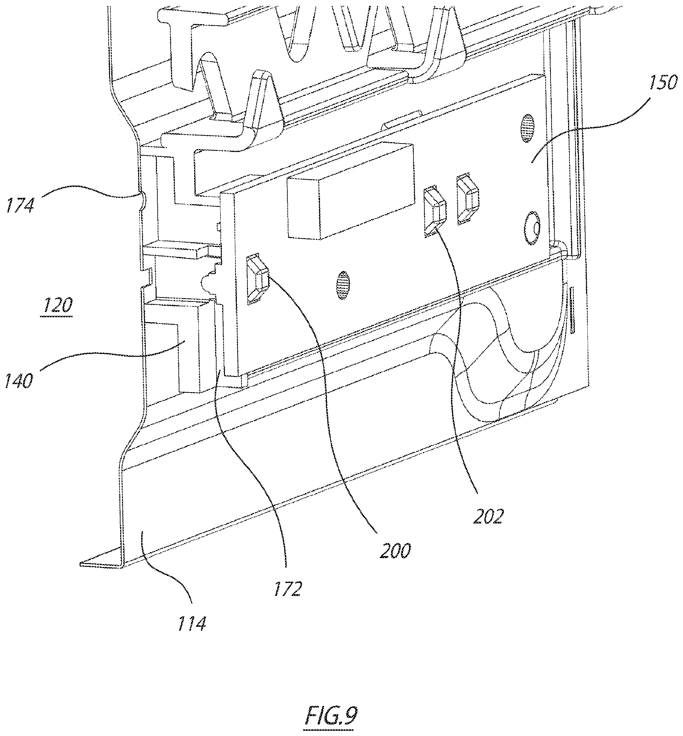

FIG. 9 shows a cut-away perspective view of the toaster of FIG. 1.

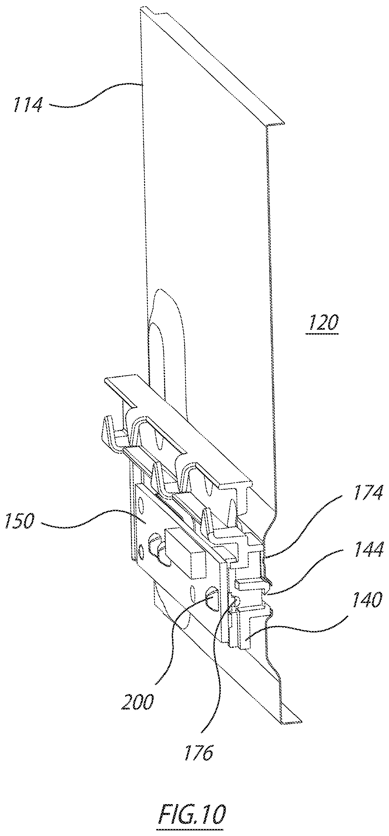

FIG. 10 shows a cut-away perspective view of the toaster of FIG. 1.
FIG. 11 shows a cut-away perspective view of the toaster of FIG. 1.

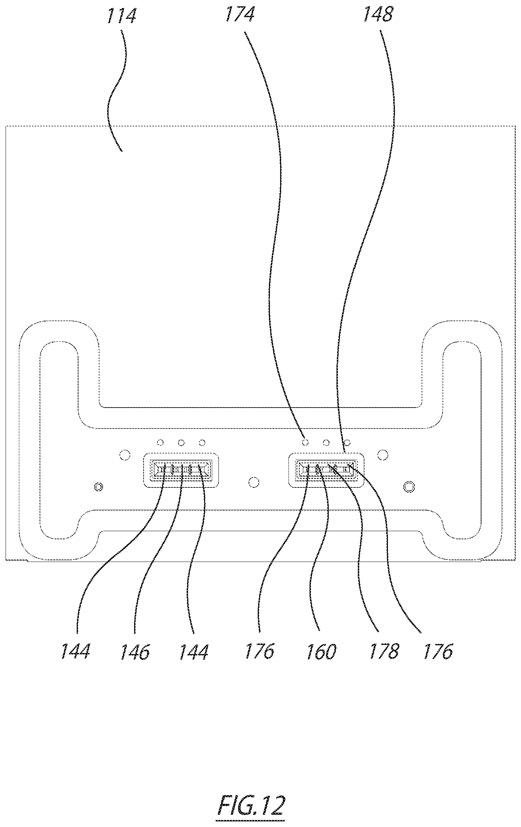

FIG. 12 shows a cut-away side view of the toaster of FIG. 1.
FIG. 13 shows an exploded perspective view of the toaster of FIG. 1.
FIG. 14 shows a cut-away perspective view of the toaster of FIG. 1.
FIG. 15 shows a detailed section view of the toaster of FIG. 1.
FIG. 16 shows a side section view of the toaster of FIG. 1.
FIG. 17 shows a cut-away perspective view of another embodiment of a heatable kitchen device according to the present invention, in this case a toaster.
FIG. 18 shows a cut-away perspective view of two different embodiments of another embodiment of a heatable kitchen device according to the present invention, in this case a toaster.
FIG. 19 shows an exploded perspective view of another embodiment of a heatable kitchen device according to the invention, in this case a toaster.
FIG. 20A is an elevation of the bracket according to the embodiment of Fig. 19.
FIG. 20B is a section view through line A-A of the bracket shown in Fig. 20A.
FIG. 21A is an elevation of the bracket and sensor assembly according to the embodiment of Fig. 19.
FIG. 21B is a section view through line B-B of Fig. 21A.

### Description of Embodiments

Where reference is made in any one or more of the accompanying drawings to steps and/or features, which have the same reference numerals, those steps and/or features have for the purposes of this description the same function(s) or operation(s), unless the contrary intention appears.

It is to be noted that the discussions contained in the "Background" section and that above relating to prior art arrangements relate to discussions of documents or devices which form public knowledge through their respective publication and/or use. Such should not be interpreted as a representation by the present inventor(s) or the patent applicant that such documents or devices in any way form part of the common general knowledge in the art.

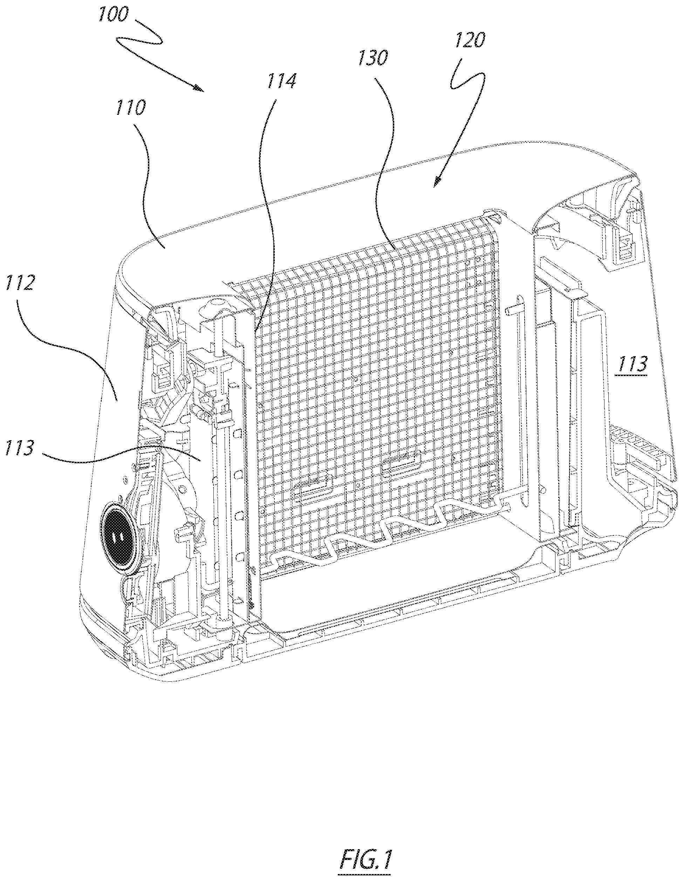

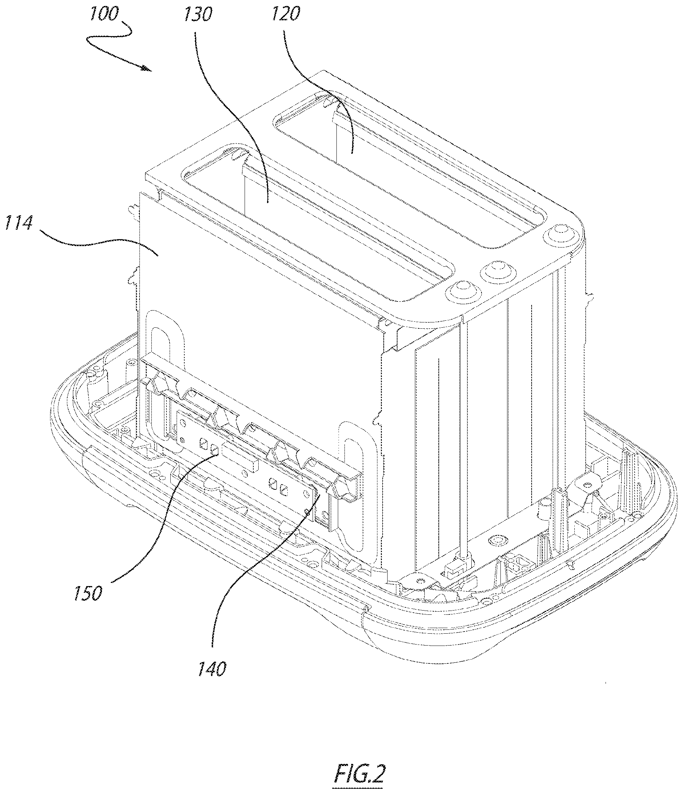

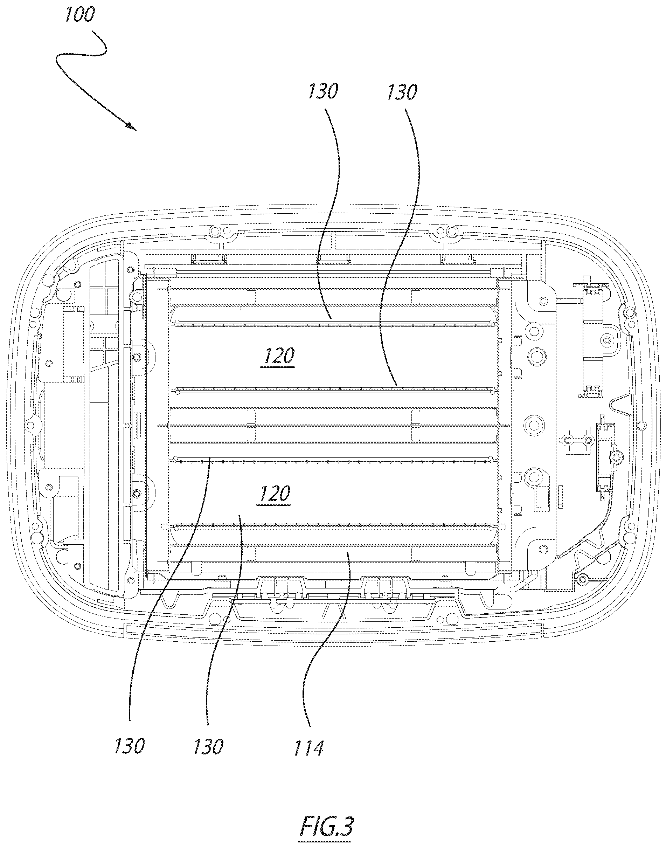

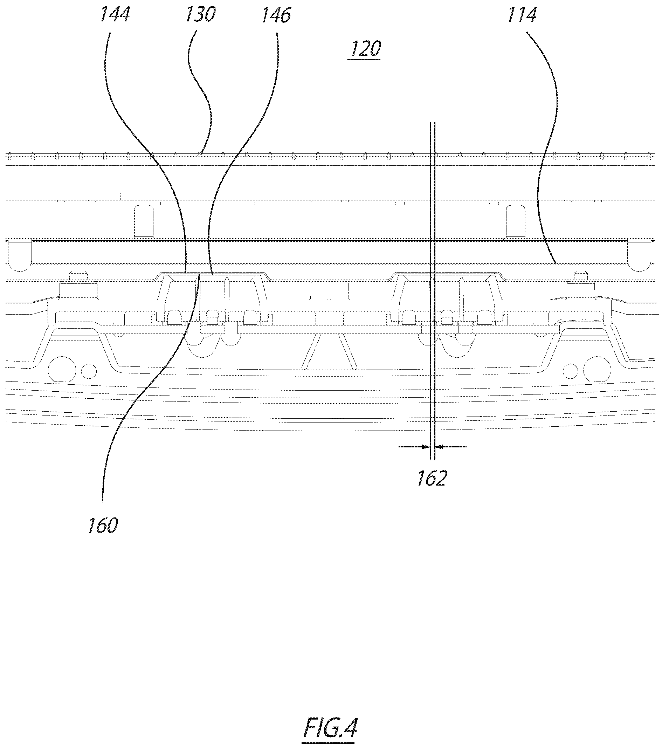

FIG. 1 shows a toaster 100, being a heatable kitchen device according to a preferred embodiment of the invention. The toaster 100 includes a housing 110 having an outer wall 112 and an inner wall 114, with a wall cavity 113 extending therebetween. As shown in FIG. 2, the inner wall 114 defines a heating area 120, in the shown preferred embodiment a pair of heating areas 120 within the housing 110 for receiving foodstuff to be heated, for example bread to be toasted. As shown in FIGS. 3 and 4, a heating element 130 is located in each heating area 120, preferable a pair of heating elements 130 is located in each heating area 120, each heating element 130 being located at opposite sides of the respective heating area 120.

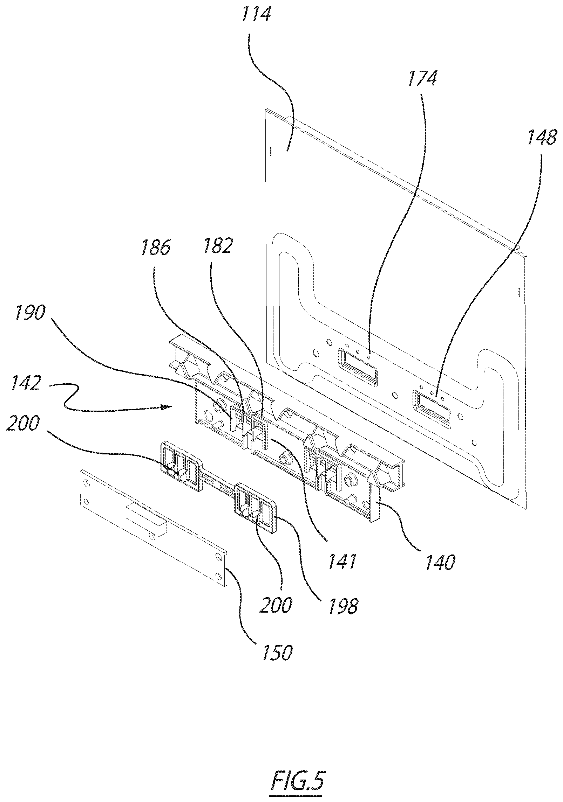

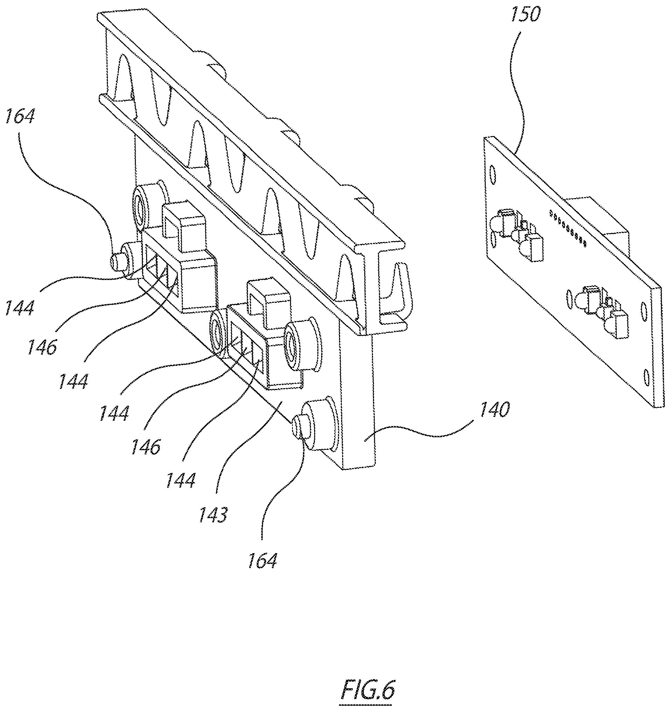

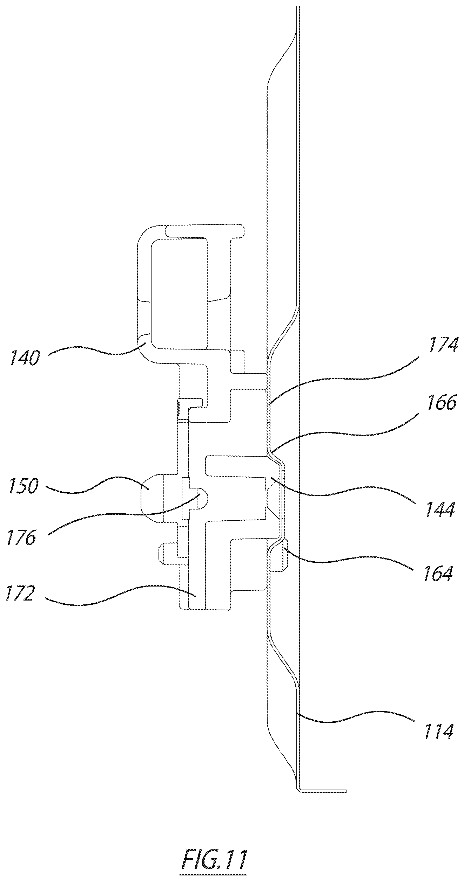

Turning to FIG. 5, the toaster 100 further includes a bracket 140, preferably manufactured from plastic material, mounted to the inner wall 114 adjacent the heating area 120. Preferably, the toaster 100 includes two brackets 140, one adjacent to each heating area 120. The bracket 140 includes, on a first side 141 facing away from the inner wall 114, a seat 142, thereby located opposite the inner wall 114. The seat 142 has a contour that conform to the physical dimensions of a sensor assembly 150 and is thereby configured to receive the sensor assembly 150. Further, as shown in FIG. 6, the bracket 140 includes a spacer 164 on a second side 143 facing the inner wall 114 to mount the bracket 140 to the inner wall 114 and be located therebetween to create an airgap 166 between the bracket 140 and the inner wall 114, best seen in FIG. 11.

Returning to FIG. 5, the bracket 140 may include a gasket 198, preferably made from silicone, located on the seat 142, so as to be located between the seat 142 and the sensor assembly 150 when the sensor assembly 150 is received in the seat 142. The gasket 198 may include extended portions 200 adapted to engage with a corresponding set of holes 202 in the sensor assembly 150 to engage and/or locate the gasket 198 on the sensor assembly 150.

Referring to FIG. 6, the bracket 140 has a first aperture 144 having an area and that extends through the bracket 140 from inner wall 114 to the seat 142. The bracket 140 also has a second aperture 146 having an area and that extends through the bracket 140 from the inner wall 114 to the seat 142. Preferably, the extended portions 200 are located between the first aperture 144 and the second aperture 146.

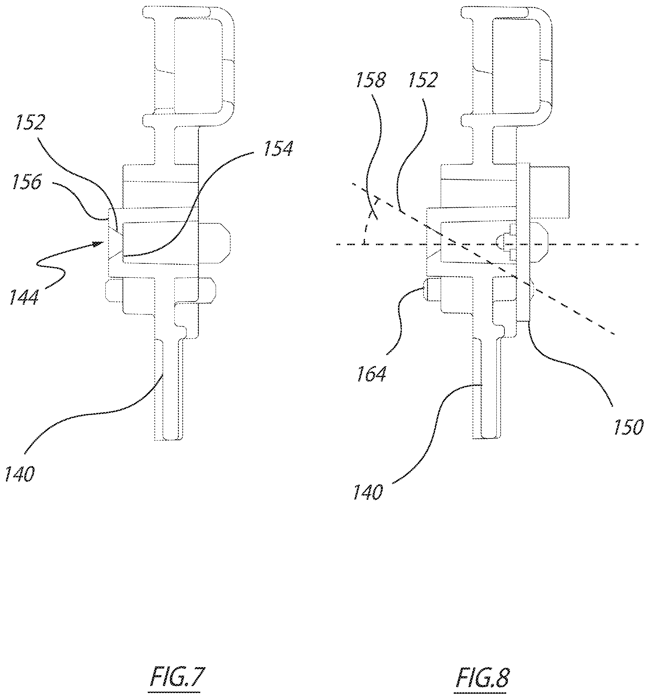

As best seen in FIGS. 7 and 8, the first and second aperture 144, 146 each have a sidewall 152. The sidewall 152 of the first and/or second aperture 144, 146 may have a slope 158, such that the area of the aperture changes from an interior side 154 of the inner wall 114 facing the foodstuff to an exterior side 156 of the inner wall 114 facing the bracket 140. The slope 158 of the sidewall 152 is preferably such that the area of the respective aperture 144, 146 at the interior side 154 is smaller than the area of the respective aperture 144, 146 at the exterior side 156.

As best seen in FIG. 4, the first and second aperture 144, 146 are adjacent and separated by a separating wall 160 having a wall thickness 162. Preferably, the wall thickness is less than 3 mm, more preferably 1.5 mm, even more preferably about 1.06 mm.

Returning to FIG. 5, the inner wall 114 has an opening 148 that is larger than the combined areas of the apertures 144, 146, and extending around the apertures 144, 146, to allow the sensor assembly 150 to emit and/or receive a test signal through the apertures 144, 146 and the opening 148 and thereby detect a property of the foodstuff. The second aperture 146 has an edge 186 extending between the first aperture 144 and the second aperture 146. Similarly, the first aperture 144 has an edge 182 that is not located between the first aperture 144 and the second aperture 146. The first aperture 144 also has a vertical edge 190 on a side opposite the second aperture 146.

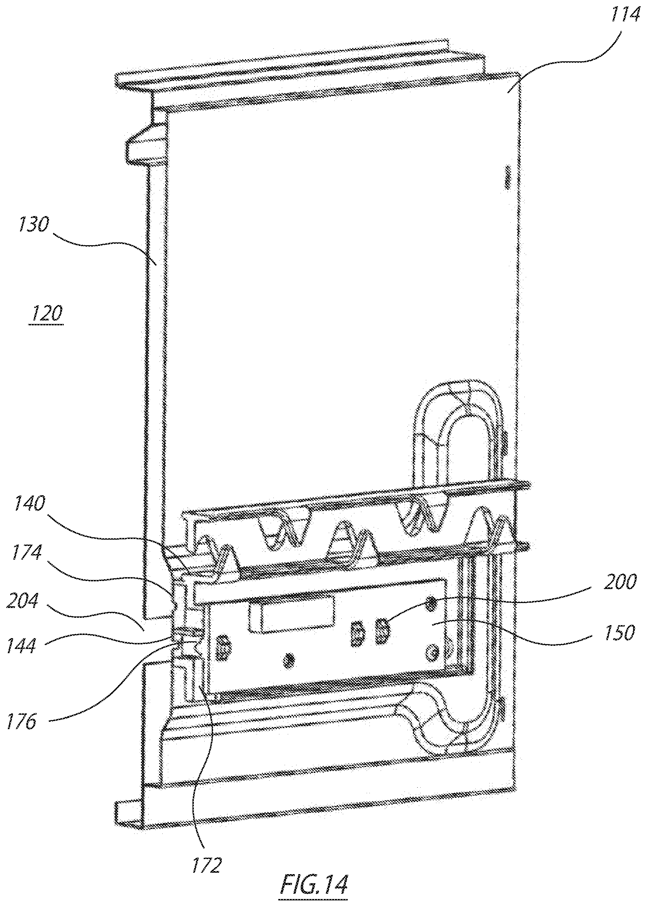

As seen in FIG. 14, the heating element 130 includes a third opening 204 that is coincident with a projection of the opening 148 and the apertures 144, 146 on the heating element 130 to allow the test signal to travel from the emitter 176 to the foodstuff in the heating area 120 and from the foodstuff to the receiver 178.

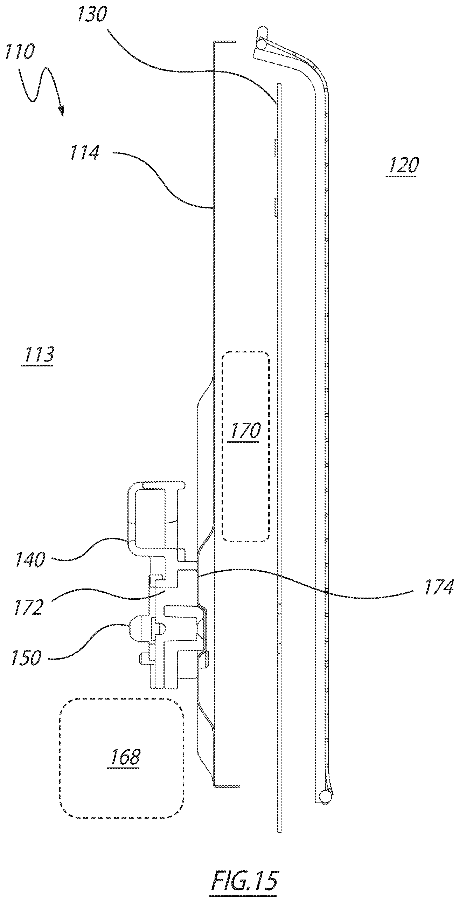

As best seen in FIG. 15, the housing 110 further includes a lower air volume 168 in the wall cavity 113, between the inner wall 114 and the outer wall 112, below the sensor assembly 150. The housing also includes an upper air volume 170 above the sensor assembly 150. In the preferred embodiment, the upper air volume 170 is located in the heating area 120. The bracket 140 includes a channel 172 connecting the lower air volume 168 to the upper air volume 170, the channel 172 being in thermal communication with the sensor assembly 150.

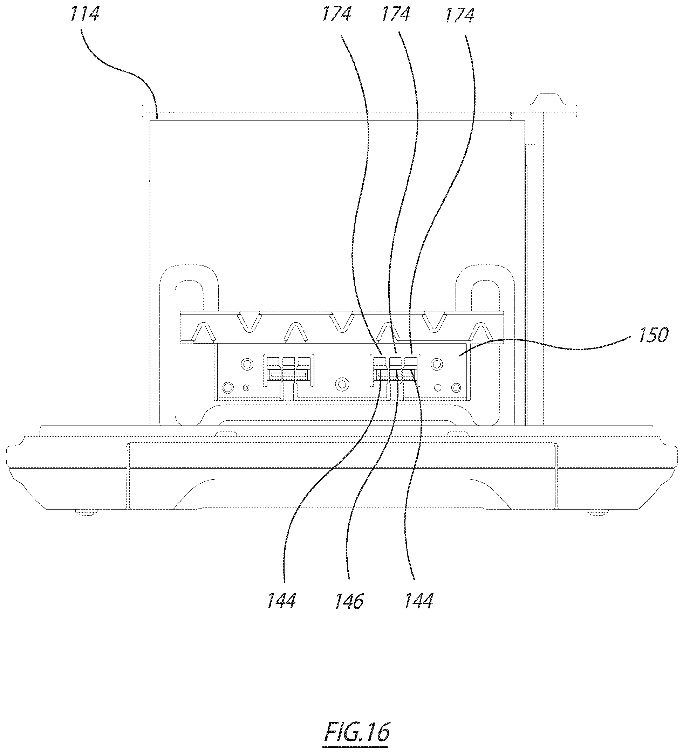

As seen in FIG. 5, the inner wall 114 has a second opening 174, preferably one second opening 174 for each aperture 144, 146 as shown in FIG. 16, above the opening 148 and preferable having a rounded shape, extending through the inner wall 114. As best seen in FIG. 15, the second opening 174 connects the heating area 120 to the channel 172 such that air from channel 172 is able to flow into the heating area 120, and subsequently the upper air volume 170, thereby drawing air, which is not heated, from the lower air volume 168 into the channel 172, thereby flowing past the sensor assembly 150. The movement of air from the channel 172 to the heating area 120 is partly driven by buoyancy of the air in the channel (being heated by the sensor assembly 150) and partly driven by the buoyancy of the air in the heating area 120, due to a decreased density of the air heated by the heating elements 130.

In some forms, the second openings 174 can be combined with each other and/or joined with the opening 148. Similarly, the second opening 174 can extend across a plurality of the sensors or a plurality of sets of sensors. Likewise, the opening 148 can extend across two or more sets of sensors 176, 178.

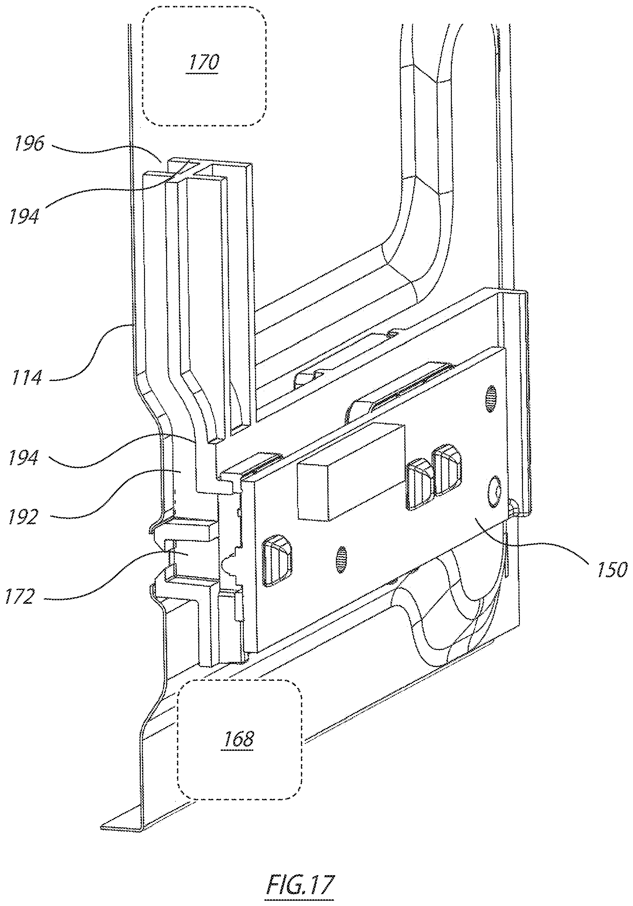

As seen in FIG. 17, the channel 172 may further include a channel extension 192 at an end of the channel 172 in the upper air volume 170. The channel extension 192 extends vertically into the upper air volume 170. The channel extension 192 has two or more side walls, preferably at least a first side wall 194 and a second side wall 196. The first side wall 194 is provided by the bracket, and the second side wall 196 is provided by the inner wall 114. Preferably, a separate channel extension 192 is provided for each of the first and second apertures 144, 146.

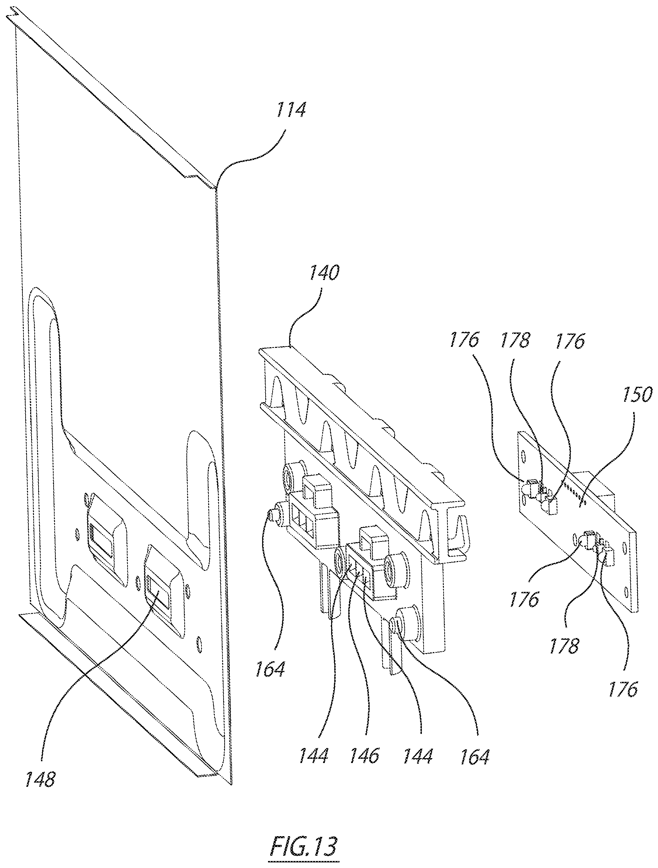

Turning to FIG. 13, the sensor assembly 150 includes an emitter 176, preferably a light emitting diode, located adjacent the first aperture 144 so as to emit the test signal through the first aperture 144 and the opening 148 to the foodstuff in the heating area 120. The test signal is reflected by the foodstuff in the heating area 120 back toward the inner wall 114. The sensor assembly 150 further includes a receiver 178 located adjacent the second aperture 146 so as to receive the test signal, when the test signal has been reflected by the foodstuff in the heating area 120 through the opening 148 and the second aperture 146. Preferably, the sensor assembly 150 includes two emitters 176 and one receiver 178 located therebetween. More preferably, the sensor assembly 150 includes two spaced sets, each set including two emitters 176 and one receiver 178 located therebetween.

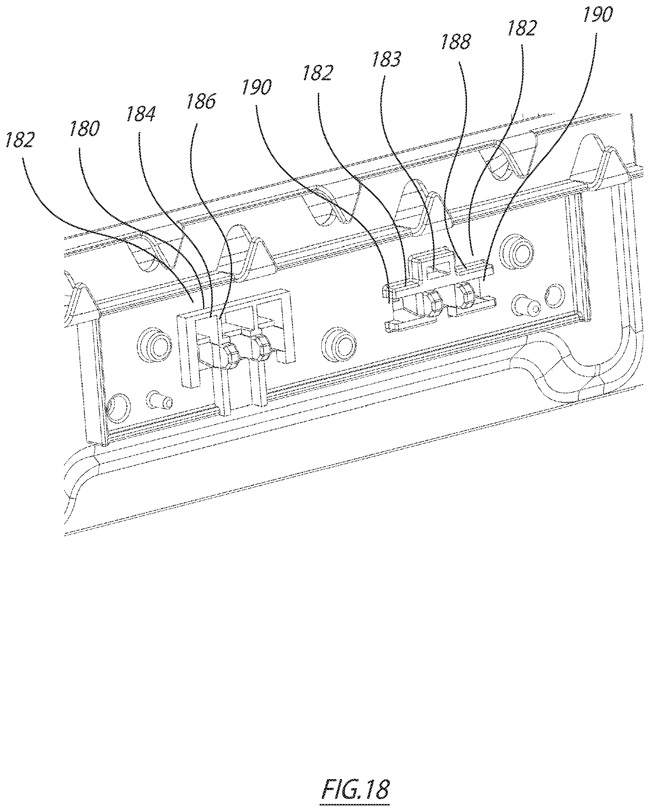

As shown in FIG. 18, the bracket 140 includes a first rib 180 on the edge 182 of the first aperture 144 (see Fig. 4). Similarly, the bracket 140 includes a second rib 184 on the edge 186 of the second aperture 146 (see Fig. 4). In a preferred embodiment, the sensor assembly 150 includes two emitters 176 and the bracket 140 includes two first apertures 144, the first apertures 144 being located on opposite sides of the second aperture 146. In another preferred embodiment also shown in FIG. 18, the second rib 184 includes a single rib 188 extending along the edge 182 of each of the two first apertures 144 as well as an edge 183 of the second aperture 146 that is colinear with the edges 182 of the first apertures 144. More preferably, the vertical edge 190 of the first apertures 144 is unobstructed by the first rib 180 and the second rib 184.

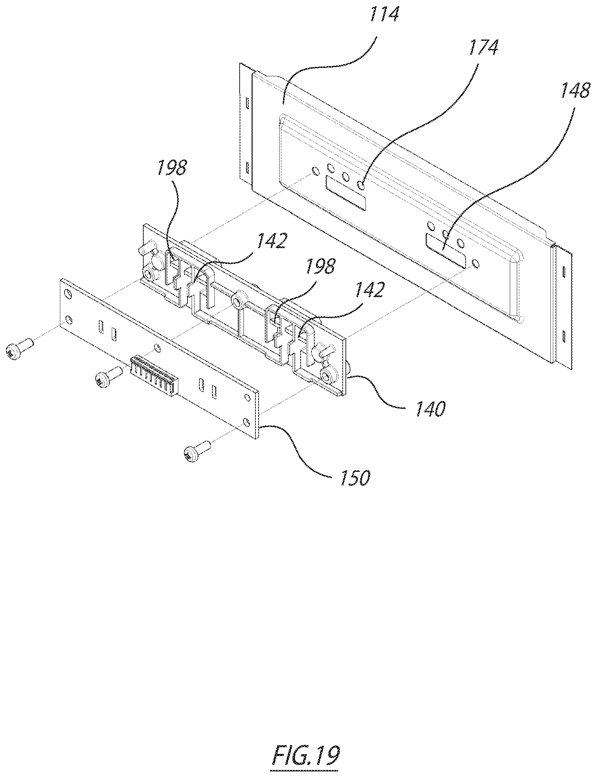

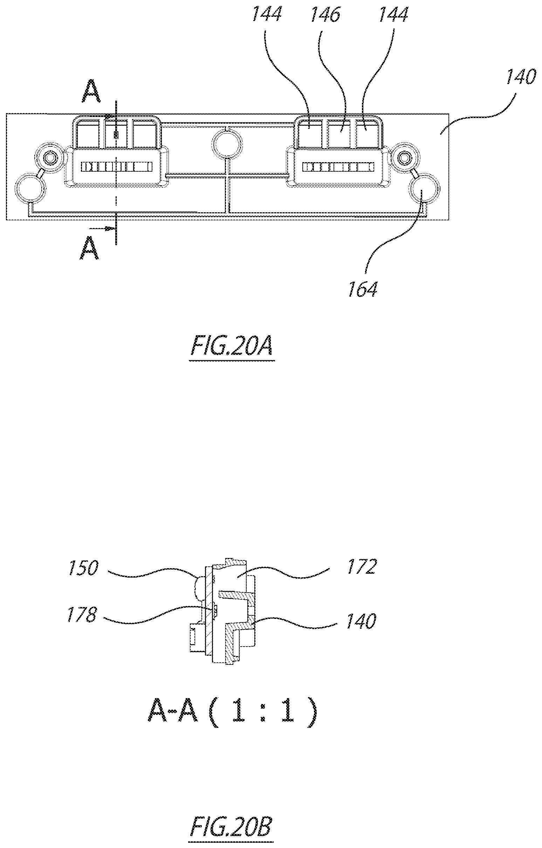

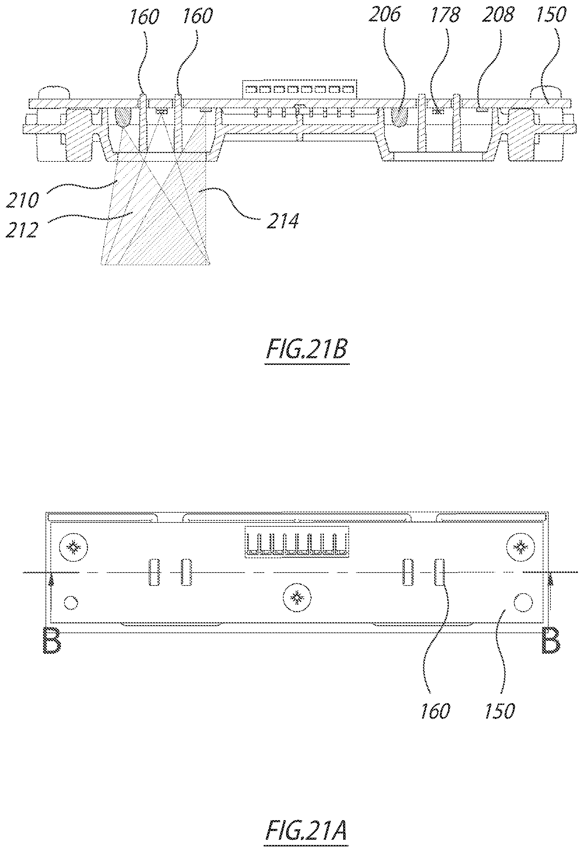

Figures 19, 20A, 20B, 21A and 21B show an embodiment in which the gasket 198 is integrated with the seat 142. Integrating the gasket 198 with the seat 142 of the bracket 140 reduces the overall number of parts and therefore production costs. Furthermore, integrating the gasket 198 and the seat 142 improves the accuracy of positioning the sensor assembly by reducing the tolerance stacking as there is one less separately assembled component. With more precise positioning of the sensor assembly relative to the first and second apertures 144, 146 there is increased reliability of achieving sensor accuracy in each toaster unit on a manufactured series. As best shown in Figures 21A and 21B, the accurate positioning of the sensor assembly 150 relative to the first and second apertures 144 and 146 together with accurate control of the thickness of the separating wall 160, provides better control of the angle of the emitted beams 210 and 214 from the emitters 206 and 208 respectively. In turn, this provides greater control of the overlapping areas of illumination of the foodstuff from the light from emitter 206 and the light 204 from emitter 208. Similarly, this provides greater control of reflected light 212 from the food stuff back to the receiver 178. Workers in this field will understand the benefits of this precision particularly in embodiments where the emitter 206 emits lights 210 having a different wavelength to the light 214 emitted by the emitter 208. For example, one emitter may emit green light while the other emits light in the infrared spectrum.

Use of the toaster 100 will now be discussed.

Foodstuff is placed in the heating area 120 and the heating elements 130 are activated to begin heating of the foodstuff. The emitter 176 emits the test signal through the first aperture 144 and the opening 148 to impinge upon the foodstuff and be reflected thereby. The reflected test signal travels back through the opening 148 and the second aperture 146 and is received by the receiver 178. The receiver 178 generates a property signal that is suitable for detecting a property of the foodstuff.

As the heating element 130, emitter 176, and receiver 178 operate, significant heat is being generated. Air that is heated by the heating element 130 in the heating area 120 rises due to buoyancy. Some air moves through the second opening 174 from the channel 172 and rises when heated due to buoyancy toward the upper air volume 170. The movement of the heated air from the second opening 174 draws further air into the channel 172 from the lower air volume 168, the air from the lower air volume 168 having a substantially lower temperature than the air in the heating area. The emitter 176 is located in the channel 172 below the second opening 174 and therefore is exposed to movement of air from the lower air volume 168 at substantially lower temperature, which absorbs heat from the emitter 176.

The channel extension 192 ensures that the heated air entering the channel 172 from the heating area 120, once mixed with air from the lower air volume 168 which is at a lower temperature, is further heated through the inner wall 114, being the second side wall 196 of the channel extension 192. The continued heating of the air in the channel extension 192 through the inner wall 114 ensures continued drawing of air at a substantially lower temperature from the lower air volume 168 into the channel 172.

When the heating element 130 stops operating, once the foodstuff has been desirably heated, the airflow through the channel 172 ceases as the buoyancy of the air provided by the heating elements subsides.

Advantages of the toaster 100 will now be discussed.

Because the first and second apertures 144, 146 are located in the bracket 140, and the opening 148 is larger than the combined areas of the apertures 144, 146, the sensor assembly 150 need only be accurately mounted to the bracket 140, compared to the greater tolerance stacking and planar location requirement of locating the apertures 144, 146 directly in the inner wall 114. This approach reduces the manufacturing cost of the inner wall 114 and results in a greater consistency of test signal paths. The use of the gasket 198 ensures proper seating of the sensor assembly 150 in the seat 142, thereby increasing the reliability of achieving sensor accuracy in each toaster unit of a manufactured series, the use of silicone for the gasket 198 increases stability of the gasket 198 in high temperatures, and increases compliance of the gasket between the seat 142 and the sensor assembly 150. Manufacturing the bracket 140 from plastic is preferable to maintain desirably tight tolerances, clean edges and improve manufacturing yield. Because the wall thickness 162 is small, the overlap of test signals from a pair of emitters 176 is larger, resulting in a greater magnitude of the test signal reflected toward the receiver 178. Use of the spacer 164 creates the airgap 166, which reduces the heat transfer from the heating element 130 to the sensor assembly 150, desirably reducing the cooling requirements of the sensor assembly 150. Because the extended portions 200 are located between the apertures 144 and the aperture 146, interference between the emitters 176 and the receiver 178 is reduced.

The slope 158 of the side walls 152 encourages a clean transmission of the test signal from the emitter 176 into the heating area 120, and also encourages wide reception of reflected test signals from the foodstuff by the receiver 178.

Use of the channel 172 provides a flow of air at a substantially lower temperature along the emitter 176 without the use of a motor, or other moving parts, desirably carrying heat away from the emitter 176. The use of the second opening 174 allows heated air from the heating area 120 to increase the magnitude of the flow of air from the lower air volume 168 to the upper air volume 170 improving cooling performance. A rounded shape of the second opening 174 provides decreased air resistance to heated air travelling through the second opening 174, increasing the flow of air from the lower air volume 168 to the upper air volume 170, improving cooling performance. The channel extension 192 yet further increases magnitude of the flow of air from the lower air volume 168 to the upper air volume 170 by increasing the heat transferred to the heated air in the channel 170, thereby increasing the buoyancy of the heated air, thereby improving cooling performance of the flow of air on the emitter 176.

Because the first rib 180 is located on the edge 182 of the first aperture 144, spillage of the test signal from the first aperture 144 is reduced. Similarly, because the second 184 rib is located on the edge 186 of the second aperture 146, spillage of an unreflected test signal directly to the receiver 176 is reduced. The use of the single rib 188 reduces spillage both from the emitter 176 and to the receiver 178. Leaving the vertical edge 190 of the aperture 144 unobstructed reduces material cost of the bracket 140, at minimal effect on spillage of the test signal to the receiver 178, as the general design of the toaster 100 makes it unlikely for there to be a reflective component located adjacent the vertical edge 190.

The embodiments and/or further developments of the above disclosure - except for example in cases of clear dependencies or inconsistent alternatives - can be applied individually or also in arbitrary combinations with one another.

### Reference Numerals:

- 100: heatable kitchen device/toaster
- 110: housing
- 112: outer wall
- 113: wall cavity
- 114: inner wall
- 120: heating area
- 130: heating element
- 140: bracket
- 141: first side
- 142: seat
- 143: second side
- 144: first aperture
- 146: second aperture
- 148: opening
- 150: sensor assembly
- 152: aperture sidewalls
- 154: interior side of inner wall
- 156: exterior side of inner wall
- 158: slope of sidewall
- 160: separating wall
- 162: wall thickness
- 164: spacer
- 166: airgap
- 168: lower air volume
- 170: upper air volume
- 172: channel
- 174: second opening
- 176: emitter
- 178: receiver
- 180: first rib
- 182: edge of first aperture
- 183: edge of second aperture that is colinear
- 184: second rib
- 186: edge of second aperture
- 188: single rib
- 190: vertical edge of apertures
- 192: channel extension
- 194: first side wall of channel extension
- 196: second side wall of channel extension
- 198: gasket
- 200: extended portions
- 202: holes in sensor assembly
- 204: third opening
- 206: emitter possibly for light of a 1^{st} wavelength
- 208: emitter possibly for light of a 2^{nd} wavelength
- 210: light emitted from emitter 206
- 212: light received by receiver 178
- 214: light emitted from emitter 208

## Claims

1. A heatable kitchen device (100) including:
a housing (110) having an outer wall (112) and an inner wall (114);
a heating area (120) within the housing for receiving foodstuff to be heated;
a heating element (130) located between the heating area (120) and the inner wall (114);
a bracket (140) mounted to the inner wall (114) adjacent the heating area (120), the bracket (140) including:
a seat (142) located opposite the inner wall (114), the seat (142) receiving a sensor assembly (150);
a first aperture (144) through the bracket (140) to the seat (142), the first aperture (144) having an area; and
a second aperture (146) through the bracket (140) to the seat (142), the second aperture (146) having an area,
wherein the inner wall (114) has an opening (148) that is larger than the combined areas of the apertures (144, 146) to allow the sensor assembly (150) to detect a property of the foodstuff,
**characterized in that** the sensor assembly (150) includes:
an emitter (176) located adjacent the first aperture (144) so as to emit a test signal to the foodstuff in the heating area (120); and
a receiver (178) located adjacent the second aperture (146) so as to receive the test signal when reflected by the foodstuff in the heating area (120),
wherein the bracket (140) includes a first rib (180) on at least one edge (182) of the first aperture (144) to reduce spillage of the test signal from the first aperture (144).

2. The heatable kitchen device (100) of claim 1, wherein the first and second aperture (144, 146) have sloped sidewalls, such that the area of the aperture (144, 446) changes from an interior side (154) of the inner wall (114) facing the foodstuff to an exterior side (156) of the inner wall (114) facing the bracket (140),
wherein the sidewalls are sloped such that the area of the apertures (144, 146) at the interior side (154) is larger than the area of the apertures (144, 146) at the exterior side (156).

3. The heatable kitchen device (100) of claim 1 or 2, wherein the bracket (140) is manufactured from plastic material, and wherein the bracket (140) further includes:
a spacer (164) to mount the bracket (140) to the inner wall (114) to create an airgap (166) between the bracket (140) and the inner wall (114).

4. The heatable kitchen device (100) of any one of the preceding claims, wherein the first aperture (144) and the second aperture (146) are adjacent and separated by a wall (160) with a wall thickness,
wherein the wall thickness is less than 3 mm, preferably less than 1.5 mm, more preferably about 1.06 mm.

5. The heatable kitchen device (100) of any one of the preceding claims, wherein the housing (110) further includes:
a lower air volume (168) between the inner wall (114) and the outer wall (112) below the sensor assembly (150); and
an upper air volume (170) above the sensor assembly (150),
wherein the bracket (140) has a channel (172) connecting the lower air volume (168) to the upper air volume (170), the channel (172) being in thermal communication with the sensor assembly (!50).

6. The heatable kitchen device (100) of claim 5, wherein the opening (148) is a first opening (148), and wherein the upper air volume (170) is located in the heating area (120) and the inner wall (114) has a second opening (174) above the first opening (148), the second opening (174) connecting the heating area (120) to the channel (172) such that air from the channel (172) is able to flow into the heating area (120) and the upper air volume (170), thereby drawing air from the lower air volume (168) into the channel (172).

7. The heatable kitchen device (100) of claim 6, wherein the second opening (174)has a rounded shape.

8. The heatable kitchen device (100) of claim 5, wherein the upper air volume (170) is located between the inner wall (114) and the outer wall (112) and the channel (172) includes:
a channel extension (192) at an end of the channel (172) extending vertically into the upper air volume (170).

9. The heatable kitchen device (100) of claim 8, wherein the channel extension (192) has two or more side walls, a first side wall (194) being provided by the bracket (140) and a second side wall (196) being provided by the inner wall (114), so that heat is transferred from the heating area (120) through the inner wall (114) to the channel extension (192).

10. The heatable kitchen device of any one of the preceding claims, wherein the bracket further includes:
a second rib (184) on at least one edge of the second aperture (146) to reduce spillage of an unreflected test signal to the receiver (178).

11. The heatable kitchen device of claim 10, wherein the sensor assembly (150) includes two emitters (176) and the bracket (140) includes two first apertures (144), the first apertures (144) being located on opposite sides of the second aperture (146), and
wherein the second rib (184) includes a single rib (188) extending along an edge of each of the two first apertures (144) and the second aperture (146).

12. The heatable kitchen device (100) of claim 11, wherein the two first apertures (144) each have a vertical edge on a side opposite the second aperture (146), the vertical edge being unobstructed by the first and second rib (180, 184).

13. The heatable kitchen device (100) of any one of the preceding claims, wherein the bracket (140) further includes:
a gasket (198) between the seat (142) and the sensor assembly (!50), and preferably the gasket (198) is integrated with the seat, wherein the gasket (198) is preferably made from silicone.

14. The heatable kitchen device (100) of claim 13, wherein the gasket (198) includes an extended portion adapted to engage a hole in the sensor assembly (150) to locate the sensor assembly (150) relative to the gasket (198), and
wherein the extended portion is located to reduce interference between an emitter (176) and a receiver (178) of the sensor assembly (150).

## Patentansprüche

1. Beheizbare Küchenvorrichtung (100), einschließlich:
eines Gehäuses (110), das eine Außenwand (112) und eine Innenwand (114) aufweist;
eines Heizbereichs (120) innerhalb des Gehäuses zum Empfangen von zu erhitzenden Lebensmitteln;
eines Heizelements (130), das zwischen dem Heizbereich (120) und der Innenwand (114) angeordnet ist;
einer Halterung (140), die an der Innenwand (114) benachbart zu dem Heizbereich (120) befestigt ist, wobei die Halterung (140) Folgendes einschließt:
einen Sitz (142), der gegenüber der Innenwand (114) angeordnet ist, wobei der Sitz (142) eine Sensorbaugruppe (150) empfängt;
einen ersten Durchlass (144) durch die Halterung (140) zu dem Sitz (142), wobei der erste Durchlass (144) einen Bereich aufweist; und
einen zweiten Durchlass (146) durch die Halterung (140) zu dem Sitz (142), wobei der zweite Durchlass (146) einen Bereich aufweist,
wobei die Innenwand (114) eine Öffnung (148) aufweist, die größer als die kombinierten Bereiche der Durchlasse (144, 146) ist, um der Sensoranordnung (150) zu ermöglichen, eine Eigenschaft des Lebensmittels zu erkennen, **dadurch gekennzeichnet, dass** die Sensorbaugruppe (150) Folgendes einschließt:
einen Emitter (176), der benachbart zu dem ersten Durchlass (144) angeordnet ist, um ein Testsignal an das Lebensmittel in dem Heizbereich (120) zu emittieren; und
einen Empfänger (178), der benachbart zu dem zweiten Durchlass (146) angeordnet ist, um das Testsignal zu empfangen, wenn es von dem Lebensmittel in dem Heizbereich (120) reflektiert wird,
wobei die Halterung (140) eine erste Rippe (180) an mindestens einer Kante (182) des ersten Durchlasses (144) einschließt, um ein Austreten des Testsignals aus dem ersten Durchlass (144) zu reduzieren.

2. Beheizbare Küchenvorrichtung (100) nach Anspruch 1, wobei der erste und der zweite Durchlass (144, 146) geneigte Seitenwände derart aufweisen, dass sich der Bereich des Durchlasses (144, 446) von einer dem Lebensmittel zugewandten Innenseite (154) der Innenwand (114) zu einer der Halterung (140) zugewandten Außenseite (156) der Innenwand (114) ändert,
wobei die Seitenwände derart geneigt sind, dass der Bereich der Durchlasse (144, 146) an der Innenseite (154) größer ist als der Bereich der Durchlasse (144, 146) an der Außenseite (156).

3. Beheizbare Küchenvorrichtung (100) nach Anspruch 1 oder 2, wobei die Halterung (140) aus Kunststoffmaterial hergestellt ist, und wobei die Halterung (140) ferner Folgendes einschließt:
einen Abstandshalter (164), um die Halterung (140) an der Innenwand (114) zu befestigen, um einen Luftspalt (166) zwischen der Halterung (140) und der Innenwand (114) zu erzeugen.

4. Beheizbare Küchenvorrichtung (100) nach einem der vorhergehenden Ansprüche, wobei der erste Durchlass (144) und der zweite Durchlass (146) benachbart und durch eine Wand (160) mit einer Wandstärke getrennt sind,
wobei die Wandstärke weniger als 3 mm, bevorzugt weniger als 1,5 mm, mehr bevorzugt etwa 1,06 mm beträgt.

5. Beheizbare Küchenvorrichtung (100) nach einem der vorhergehenden Ansprüche, wobei das Gehäuse (110) ferner Folgendes einschließt:
ein unteres Luftvolumen (168) zwischen der Innenwand (114) und der Außenwand (112) unterhalb der Sensorbaugruppe (150); und
ein oberes Luftvolumen (170) über der Sensorbaugruppe (150),
wobei die Halterung (140) einen Kanal (172) aufweist, der das untere Luftvolumen (168) mit dem oberen Luftvolumen (170) verbindet, wobei der Kanal (172) in thermischer Kommunikation mit der Sensorbaugruppe (150) steht.

6. Beheizbare Küchenvorrichtung (100) nach Anspruch 5, wobei die Öffnung (148) eine erste Öffnung (148) ist, und wobei das obere Luftvolumen (170) in dem Heizbereich (120) angeordnet ist und die Innenwand (114) eine zweite Öffnung (174) über der ersten Öffnung (148) aufweist, wobei die zweite Öffnung (174) den Heizbereich (120) mit dem Kanal (172) derart verbindet, dass Luft aus dem Kanal (172) in der Lage ist, in den Heizbereich (120) und das obere Luftvolumen (170) zu strömen, wodurch Luft aus dem unteren Luftvolumen (168) in den Kanal (172) gesaugt wird.

7. Beheizbare Küchenvorrichtung (100) nach Anspruch 6, wobei die zweite Öffnung (174) eine abgerundete Form aufweist.

8. Beheizbare Küchenvorrichtung (100) nach Anspruch 5, wobei das obere Luftvolumen (170) zwischen der Innenwand (114) und der Außenwand (112) angeordnet ist und der Kanal (172) Folgendes einschließt:
eine Kanalerstreckung (192) an einem Ende des Kanals (172), die sich vertikal in das obere Luftvolumen (170) erstreckt.

9. Beheizbare Küchenvorrichtung (100) nach Anspruch 8, wobei die Kanalerstreckung (192) zwei oder mehrere Seitenwände aufweist, wobei eine erste Seitenwand (194) durch die Halterung (140) bereitgestellt ist und eine zweite Seitenwand (196) durch die Innenwand (114) bereitgestellt ist, sodass Wärme von dem Heizbereich (120) durch die Innenwand (114) zu der Kanalerstreckung (192) übertragen wird.

10. Beheizbare Küchenvorrichtung nach einem der vorhergehenden Ansprüche, wobei die Halterung ferner Folgendes einschließt:
eine zweite Rippe (184) an mindestens einem Rand des zweiten Durchlasses (146), um das Austreten eines unreflektierten Testsignals an den Empfänger (178) zu reduzieren.

11. Beheizbare Küchenvorrichtung nach Anspruch 10, wobei die Sensorbaugruppe (150) zwei Emitter (176) einschließt und die Halterung (140) zwei erste Durchlasse (144) einschließt, wobei die ersten Durchlasse (144) auf gegenüberliegenden Seiten des zweiten Durchlasses (146) angeordnet sind, und
wobei die zweite Rippe (184) eine einzelne Rippe (188) einschließt, die sich entlang einer Kante jeder der zwei ersten Durchlasse (144) und des zweiten Durchlasses (146) erstreckt.

12. Beheizbare Küchenvorrichtung (100) nach Anspruch 11, wobei die zwei ersten Durchlasse (144) jeweils eine vertikale Kante auf einer dem zweiten Durchlass (146) gegenüberliegenden Seite aufweisen, wobei die vertikale Kante durch die erste und zweite Rippe (180, 184) nicht blockiert ist.

13. Beheizbare Küchenvorrichtung (100) nach einem der vorhergehenden Ansprüche, wobei die Halterung (140) ferner Folgendes einschließt:
eine Dichtung (198) zwischen dem Sitz (142) und der Sensorbaugruppe (150), und bevorzugt ist die Dichtung (198) in den Sitz integriert, wobei die Dichtung (198) bevorzugt aus Silikon hergestellt ist.

14. Beheizbare Küchenvorrichtung (100) nach Anspruch 13, wobei die Dichtung (198) einen erstreckten Abschnitt einschließt, der dazu ausgebildet ist, in ein Loch in der Sensorbaugruppe (150) einzugreifen, um die Sensorbaugruppe (150) relativ zu der Dichtung (198) anzuordnen, und
wobei der erstreckte Abschnitt dazu angeordnet ist, eine Interferenz zwischen einem Emitter (176) und einem Empfänger (178) der Sensorbaugruppe (150) zu reduzieren.

## Revendications

1. Dispositif de cuisine chauffant (100) comportant :
un boîtier (110) présentant une paroi externe (112) et une paroi interne (114) ;
une zone de chauffage (120) à l'intérieur du boîtier pour recevoir la denrée alimentaire à chauffer ;
un élément chauffant (130) situé entre la zone de chauffage (120) et la paroi interne (114) ;
un support (140) monté sur la paroi interne (114) adjacente à la zone de chauffage (120), le support (140) comportant :
un siège (142) situé à l'opposé de la paroi interne (114), le siège (142) recevant un ensemble capteur (150) ;
une première ouverture (144) à travers le support (140) vers le siège (142), la première ouverture (144) présentant une zone ; et
une seconde ouverture (146) à travers le support (140) vers le siège (142), la seconde ouverture (146) présentant une surface,
dans lequel la paroi interne (114) présente une ouverture (148) qui est plus grande que les surfaces combinées des ouvertures (144, 146) pour permettre à l'ensemble capteur (150) de détecter une propriété de la denrée alimentaire, **caractérisé en ce que** l'ensemble capteur (150) comporte :
un émetteur (176) situé adjacent à la première ouverture (144) de manière à émettre un signal d'essai vers la denrée alimentaire dans la zone de chauffage (120) ; et
un récepteur (178) situé à côté de la seconde ouverture (146) de manière à recevoir le signal d'essai lorsqu'il est réfléchi par la denrée alimentaire dans la zone de chauffage (120),
dans lequel le support (140) comporte une première nervure (180) sur au moins un bord (182) de la première ouverture (144) pour réduire le déversement du signal d'essai depuis la première ouverture (144).

2. Dispositif de cuisine chauffant (100) selon la revendication 1, dans lequel la première et la seconde ouverture (144, 146) présentent des parois latérales inclinées, de telle sorte que la zone de l'ouverture (144, 446) passe d'un côté intérieur (154) de la paroi interne (114) faisant face à la denrée alimentaire vers un côté extérieur (156) de la paroi interne (114) faisant face au support (140),
dans lequel les parois latérales sont inclinées de telle sorte que la zone des ouvertures (144, 146) au niveau du côté intérieur (154) est plus grande que la zone des ouvertures (144, 146) au niveau du côté extérieur (156).

3. Dispositif de cuisine chauffant (100) selon la revendication 1 ou 2, dans lequel le support (140) est fabriqué en matière plastique, et dans lequel le support (140) comporte en outre :
un séparateur (164) pour monter le support (140) sur la paroi interne (114) afin de créer un espace d'air (166) entre le support (140) et la paroi interne (114).

4. Dispositif de cuisine chauffant (100) selon l'une quelconque des revendications précédentes, dans lequel la première ouverture (144) et la seconde ouverture (146) sont adjacentes et séparées par une paroi (160) avec une épaisseur de paroi,
dans lequel l'épaisseur de paroi est inférieure à 3 mm, de préférence inférieure à 1,5 mm, plus préférablement d'environ 1,06 mm.

5. Dispositif de cuisine chauffant (100) selon l'une quelconque des revendications précédentes, dans lequel le boîtier (110) comporte en outre :
un volume d'air inférieur (168) entre la paroi interne (114) et la paroi externe (112) en dessous de l'ensemble capteur (150) ; et
un volume d'air supérieur (170) au-dessus de l'ensemble capteur (150),
dans lequel le support (140) présente un canal (172) reliant le volume d'air inférieur (168) au volume d'air supérieur (170), le canal (172) étant en communication thermique avec l'ensemble capteur (150).

6. Dispositif de cuisine chauffant (100) selon la revendication 5, dans lequel l'ouverture (148) est une première ouverture (148), et dans lequel le volume d'air supérieur (170) est situé dans la zone de chauffage (120) et la paroi interne (114) présente une seconde ouverture (174) au-dessus de la première ouverture (148), la seconde ouverture (174) reliant la zone de chauffage (120) au canal (172) de telle sorte que l'air provenant du canal (172) peut s'écouler dans la zone de chauffage (120) et le volume d'air supérieur (170), aspirant ainsi de l'air provenant du volume d'air inférieur (168) dans le canal (172).

7. Dispositif de cuisine chauffant (100) selon la revendication 6, dans lequel la seconde ouverture (174) présente une forme arrondie.

8. Dispositif de cuisine chauffant (100) selon la revendication 5, dans lequel le volume d'air supérieur (170) est situé entre la paroi interne (114) et la paroi externe (112) et le canal (172) comporte :
une extension de canal (192) au niveau d'une extrémité du canal (172) s'étendant verticalement dans le volume d'air supérieur (170).

9. Dispositif de cuisine chauffant (100) selon la revendication 8, dans lequel l'extension de canal (192) présente deux parois latérales ou plus, une première paroi latérale (194) étant fournie par le support (140) et une seconde paroi latérale (196) étant fournie par la paroi interne (114), de sorte que la chaleur est transférée de la zone de chauffage (120) à travers la paroi interne (114) vers l'extension de canal (192).

10. Dispositif de cuisine chauffant selon l'une quelconque des revendications précédentes, dans lequel le support comporte en outre :
une seconde nervure (184) sur au moins un bord de la seconde ouverture (146) pour réduire le déversement d'un signal d'essai non réfléchi vers le récepteur (178).

11. Dispositif de cuisine chauffant selon la revendication 10, dans lequel l'ensemble capteur (150) comporte deux émetteurs (176) et le support (140) comporte deux premières ouvertures (144), les premières ouvertures (144) étant situées sur des côtés opposés de la seconde ouverture (146), et
dans lequel la seconde nervure (184) comporte une nervure unique (188) s'étendant le long d'un bord de chacune des deux premières ouvertures (144) et de la seconde ouverture (146).

12. Dispositif de cuisine chauffant (100) selon la revendication 11, dans lequel les deux premières ouvertures (144) présentent chacune un bord vertical sur un côté opposé à la seconde ouverture (146), le bord vertical n'étant pas obstrué par la première et la seconde nervure (180, 184).

13. Dispositif de cuisine chauffant (100) selon l'une quelconque des revendications précédentes, dans lequel le support (140) comporte en outre :
un joint d'étanchéité (198) entre le siège (142) et l'ensemble capteur (150), et de préférence le joint d'étanchéité (198) est intégré au siège, dans lequel le joint d'étanchéité (198) est de préférence en silicone.

14. Dispositif de cuisine chauffant (100) selon la revendication 13, dans lequel le joint d'étanchéité (198) comporte une partie étendue conçue pour venir en prise avec un trou dans l'ensemble capteur (150) pour localiser l'ensemble capteur (150) par rapport au joint d'étanchéité (198), et
dans lequel la partie étendue est située de manière à réduire l'interférence entre un émetteur (176) et un récepteur (178) de l'ensemble capteur (150).
